# EUROPÄISCHE PATENTSCHRIFT

(11) **EP 1 296 986 B1**
(45) Veröffentlichungstag und Bekanntmachung des Hinweises auf die Patenterteilung: **10.08.2011**
(21) Anmeldenummer: 01957901.0
(22) Anmeldetag: 28.06.2001
(51) Int. Cl.: C07D 487/04, A61K 31/519, A61P 9/00, A61P 15/10

(54) **5-AMINOALKYL-PYRAZOLO[4,3-D]PYRIMIDINE MIT PHOSPHODIESTERASE V INHIBIERENDER WIRKUNG**
5-AMINOALKYL-PYRAZOLO[ 4,3-D]PYRIMIDINES WITH A PHOSPHODIESTERASE V-INHIBITING EFFECT
5-AMINOALKYLE-PYRAZOLO[4,3-D]PYRIMIDINES A EFFET INHIBANT LA PHOSPHOSDIESTERASE V

(30) Priorität: 29.06.2000 DE 10031584
(43) Veröffentlichungstag der Anmeldung: 02.04.2003
(73) Patentinhaber: Merck Patent GmbH, 64293 Darmstadt (DE)
(72) Erfinder: JONAS, Rochus, 64291 Darmstadt (DE); SCHELLING, Pierre, 64367 Mühltal (DE); CHRISTADLER, Maria, 63322 Rödermark (DE); BEIER, Norbert, 64354 Reinheim (DE)
(86) Internationale Anmeldenummer: PCT/EP2001/007378
(87) Internationale Veröffentlichungsnummer: WO 2002/000660

(56) Entgegenhaltungen:
- EP-A- 0 349 239
- EP-A- 0 579 496
- WO-A-01/18004
- WO-A-96/16657
- WO-A-98/17668
- WO-A-99/28325
- US-A- 6 001 830
- CZARNIECKI,M. ET AL.: "Inhibitors of Types I and V Phosphodiesterase : Elevation of cGMP as a Therapeutic Strategy " ANNU.REP.MED.CHEM., Bd. 31, 1996, Seiten 61-70, XP002180272 NEW YORK
- DUMAITRE B ET AL: "SYNTHESIS AND CYCLIC GMP PHOSPHODIESTERASE INHIBITORY ACTIVITY OF A SERIES OF 6-PHENYLPYRAZOLOU3,4-DPYRIMIDONES" JOURNAL OF MEDICINAL CHEMISTRY, AMERICAN CHEMICAL SOCIETY. WASHINGTON, US, Bd. 39, Nr. 8, 1996, Seiten 1635-1644, XP000651134 ISSN: 0022-2623

## Beschreibung

Die vorliegende Erfindung betrifft 5-Aminoalkyl-pyrazolo[4,3-d]pyrimidine der allgemeinen Formel (I) 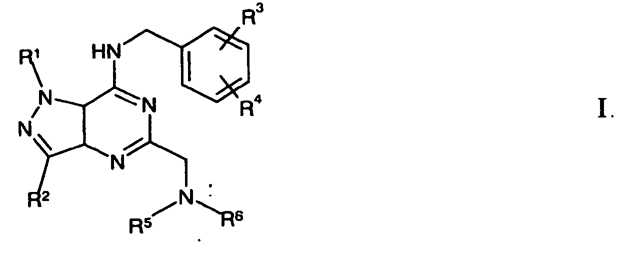 in der die Reste R¹ bis R⁶ die im Text angegebene Bedeutung haben, sowie deren Verwendung als Arzneimittel, insbesondere als c-GMP-Phosphodiesteraseinhibitor.

Substanzen mit c-GMP-Phosphodiesterase-hemmenden Eigenschaften sind seit mehreren Jahren bekannt. Sie dienen bei krankhaft erhöhtem cyclo-Guanosinmonophosphat (c-GMP)-Spiegel zu dessen Senkung. Dadurch werden die bei einem erhöhtem c-GMP-Spiegel auftretenden Symptome wie Entzündungshemmung und -verhinderung und Muskelentspannung unterdrückt bzw. verhindert. c-GMP-Phosphodiesterasehemmer kommen insbesondere zur Behandlung von Herz-Kreislaufkrankheiten sowie von Potenzstörungen zum Einsatz.

Es gibt verschiedene Molekül-Verbindungsklassen, die für ihre c-GMP-Phosphodiesterase-hemmenden Eigenschaften bekannt sind.

Dies sind zum einem Chinazoline, die beispielsweise in J.Med.Chem.36, Seite 3765 ff (1993) und in J.Med.Chem. 37, Seite 7106 ff. (1994) beschrieben sind.

Zum anderen eignen sich auch Pyrazolopyrimidinone, die in der WO 94/28902 beschrieben sind. Hier wird auch die Verwendung der Substanzklasse zur Behandlung von Impotenz offenbart.

Andere heterocyclische Phosphodiesterase V - Inhibitoren sind beschrieben in WO 01/18004, WO 99/28325, WO 98/17668, Annu. Rep. Med. Chem. 31, 61 (1996), EP 0 579 496 A1, US 6,001,830, WO 96/16657, J. Med. Chem. 39, 1635 (1996), EP 0 349 239.

Die Verwendung von Thieno[2,3-d]pyrimidinen zur Behandlung von Herz-Kreislauferkrankungen und Impotenz ist in der deutschen Anmeldung mit dem Aktenzeichen 196 44 228.1 offenbart.

Schließlich ist in der deutschen Anmeldung mit dem Aktenzeichen 199 42 474.8 die Verwendung von Pyrazolo[4,3-d]pyrimidinen mit der allgemeinen Formel 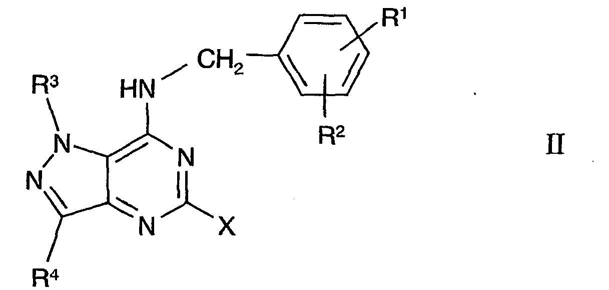 beschrieben. Die Substituenten R¹ bis R⁴ stehen dabei für eine Hydroxygruppe, verschiedene Alkyl-, Alkoxy- oder Halogengruppen oder Wasserstoff. X ist entweder eine Cycloalkylgruppe oder Cycloakylengruppe mit 5 bis 12 C-Atomen, eine Phenyl- oder Phenylmethylgruppe oder eine lineare oder verzweigte Alkylengruppe mit 1 bis 10 C-Atomen, in der ein oder zwei CH₂-Gruppen durch -CH=CH-Gruppen ersetzt sein können, und wobei sämtliche oben für den Substituenten X definierten Gruppen einfach mit einer -COOH-, C(O)O(C₁-C₆-Alkyl)-, -C(O)NH₂-, -C(O)NH(C₁-C₆-Alkyl), -C(O)N(C₁-C₆-Alkyl)₂ oder -CN-Gruppe substituiert sind.

Es ist die Aufgabe der vorliegenden Erfindung, neue Verbindungen bereitzustellen, die als Arzneimittel verwendet werden, wobei insbesondere eine Verwendung als c-GMP-Phosphodiesteraseinhibitor erwünscht ist. Diese Aufgabe wird gelöst durch eine Verbindung der Formel 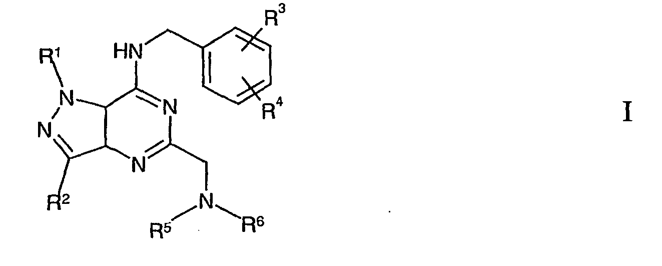 wobei
R¹, R² entweder gleich oder verschieden sind und unabhängig voneinander Wasserstoff oder eine C₁-C₈-Alkylgruppe bedeuten,
R³, R⁴ entweder gleich oder verschieden sind und unabhängig voneinander Wasserstoff, eine Hydroxylgruppe, eine lineare oder verzweigte C₁-C₈-Alkylgruppe, eine C₁-C₈-Alkoxygruppe oder Halogen bedeuten, oder zusammen mit den C-Atomen, an die sie gebunden sind, einen 5-8-gliedrigen Ring bilden, der neben Kohlenstoffatomen gegebenenfalls ein oder mehrere Sauerstoffatome enthalten kann,
R⁵, R⁶ gleich oder verschieden sein können und unabhängig voneinander Wasserstoff, eine lineare oder verzweigte C₁-C₈₋Alkylgruppe, die mit einer oder mehreren Hydroxyl-, C₁-C₈-Alkoxy-, Amin-, Mono(C₁-C₈-Alkyl)amin-, Di(C₁-C₈-Alkyl)amin-, Nitrilo-, N-Morpholino-, Phenyl-, Benzodioxol- oder Pyridylgruppen substituiert sein kann, oder eine C₄-C₇-Cycloalkylgruppe bedeuten, oder gemeinsam mit dem Stickstoffatom, an die sie gebunden sind, einen gesättigten heterocyclischen Ring bilden, der gegebenenfalls eines oder mehrere weitere Stickstoff- und/oder Sauerstoffatome enthält und der mit einer oder mehreren C₁-C₈-Alkyl-, Hydroxy-, C₁-C₈-Alkoxy-, C₁-C₈-Alkylol-, C₁-C₆-Oligohydroxyalkyl-, Amino- Mono(C₁-C₈-Alkyl)amino-, Di(C₁-C₈-Alkyl)amino-, -SO₂R⁷- oder -C(O)R⁷-Gruppen substituiert ist,
R⁷ eine C₁-C₈-Alkyl-, C₁-C₈-Fluoralkyl-, eine gegebenenfalls durch Alkyl-, Halogen- oder Nitrilgruppen substituierte Phenylgruppe oder eine Benzodioxolgruppe ist.

Es wurde gefunden, daß die 5-Aminoalkyl-pyrazolo[4,3-d]pyrimidine der Formel (I) sowie deren physiologisch verträgliche Salze vorteilhafte pharmakologische Eigenschaften aufweisen.

Insbesondere zeigen die Moleküle der allgemeinen Formel (I) eine spezifische Inhibierung der c-GMP-Phosphodiesterase. Daher sind diese Verbindungen insbesondere zur Behandlung von Erkrankungen des Herz-Kreislaufsystems sowie der Behandlung und der Therapie von Potenzstörungen, die in Form von erektiler Dysfunktion auftreten, geeignet.

Die Ermittlung der biologischen Aktivität der erfindungsgemäßen Verbindungen (I) geschieht beispielsweise nach der Methode, die in der WO 93/06104 beschrieben ist. Dabei wird die Affinität der erfindungsgemäßen Verbindungen für c-GMP- und c-AMP-Phosphodiesterase durch die Ermittlung der IC₅₀-Werte bestimmt. Der IC₅₀-Wert ist dabei die Konzentration des Inhibitors, die zu einer 50 %-igen Inhibierung der Enzymaktivität benötigt wird. Die dabei verwendeten Phosphodiesterasen können nach bekannten Methoden isoliert werden, die beispielsweise von W.J. Thompson et al. in Biochem. 10, Seiten 311 ff (1971) beschrieben werden. Die Durchführung der Versuche kann etwa nach der modifizierten "batch"-Methode von W.J. Thompson und M.M. Appleman, beschrieben in Biochem. 18, Seiten 5228 ff (1979), erfolgen.

Die Wirksamkeit der erfindungsgemäßen Verbindungen der Formel (I) bei der Behandlung und Therapie von Potenzstörungen wurde durch Inhibierung der Phenylephedrin-induzierten Kontraktionen in Corpus Cavernosum-Präparationen von Hasen nachgewiesen. Dabei erfolgt der Nachweis der biologischen Aktivität vorteilhafterweise nach der Methode, die von F. Holmquist et al. in J. Urol., 150, Seiten 1310 bis 1350 (1993) beschrieben wird.

Vorteilhafte Resultate konnten erhalten werden, wenn Verbindungen der allgemeinen Formel (I) verwendet wurden, in denen die Reste R¹ bis R⁵ die nachfolgend angegebene Bedeutung haben.

Die Reste R¹, R² sind verschieden und ausgewählt aus der Gruppe bestehend aus Wasserstoff und linearen oder verzweigten (C₁-C₄)-Alkylgruppen, wobei R¹ insbesondere eine Methyl- oder Ethylgruppe und R² insbesondere eine Propyl- oder Butylgruppe ist.

Die Reste R³ und R⁴ können gleich oder verschieden sein und befinden sich in den Positionen 3 und 4 des Phenylrings; sie bedeuten unabhängig voneinander Wasserstoff, eine lineare oder verzweigte (C₁-C₆)-Alkylgruppe, eine lineare oder verzweigte (C₁-C₆)-Alkoxygruppe oder Halogen, oder bilden zusammen eine Propylen-, Butylen-, Pentylen-, Ethylenoxy-, Methylenoxy- oder Ethylendioxygruppe.

Die Reste R⁵ und R⁶ können gleich oder verschieden sein und bedeuten unabhängig voneinander Wasserstoff oder eine C₁-C₆-Alkylgruppe, die unsubstituiert ist oder mit einer oder mehreren Hydroxy-, Methoxy-, Ethoxy-, Nitril-, Methylamin-, Ethylamin-, Dimethylamin-, Diethylamin-, Pyridyl-, Benzodioxol- oder N-Morpholinogruppen substituiert ist, oder eine Cyclopentyl- oder Cyclohexylgruppe, oder R⁵, R⁶ bilden mit dem Stickstoffatom, an das sie gebunden sind, einen Piperidinyl- oder Piperazinylring, der gegebenenfalls mit einer oder mehreren Hydroxy-, Hydroxycarbonyl-, C₁-C₂-Alkylamin-, -SO₂-R⁷ oder -C(O)-R⁷-Gruppen substituiert ist, wobei R⁷ eine C₁-C₃-Alkyl-, eine C₁-C₃-Fluoralkyl-, eine durch eine oder mehrere Alkyl- oder Nitrilgruppen substituierte Phenylgruppe oder eine Benzodioxolgruppe ist.

Die erfindungsgemäßen Verbindungen eignen sich zu Anwendung als Arzneimittel, wobei sowohl Human- als auch Veterinärmedikamente hergestellt werden können.

Bei diesen Anwendungen werden die erfindungsgemäßen Verbindungen häufig in Form ihrer physiologisch verträglichen Salze eingesetzt. Als solche eignen sich allgemein Metallsalze, beispielsweise Alkali- und Erdalkalimetallsalze, und Ammoniumsalze, beispielsweise von Ammoniak selbst oder von organischen Aminen.

Eine andere, bei den erfindungsgemäßen Verbindungen bevorzugte Form von Salzen sind Säureadditionssalze. Diese lassen sich mit den üblichen, einem Fachmann bekannten Verfahren darstellen, beispielsweise durch Umsetzen der erfindungsgemäßen Verbindungen mit der jeweiligen Säure in einem inerten Lösungsmittel und anschließendem Isolieren des Salzes, beispielsweise durch Eindampfen. Beispiele für Säuren, die physiologisch unbedenkliche Salze liefern, sind zum einem anorganische Säuren, wie beispielsweise Schwefelsäure, Salpetersäure, Halogenwasserstoffsäure, beispielsweise Chlorwasserstoffsäure oder Bromwasserstoffsäure, Phosphorsäuren, beispielsweise Orthophosphorsäure, oder Sulfaminsäure.

Zu anderen bilden auch organische Säuren geeignete Salze. Dies sind beispielsweise Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Zitronensäure, Gluconsäure, Ascorbinsäure, Nikotinsäure, iso-Nikotinsäure, Methan- und Ethansulfonsäure, Ethandisulfonsäure, 2-Hydroxyethansulfonsäure, Benzolsulfonsäure, p-Toluolsulfonsäure, Naphthalin-mono- und -disulfonsäuren, und Laurylschwefelsäure.

Zur Verwendung als Arzneimittel werden die erfindungsgemäßen Verbindungen bzw. deren physiologisch unbedenkliche Salze zu geeigneten pharmazeutischen Zubereitungen konfektioniert. Dabei werden sie mit mindestens einem geeigneten Träger oder einem Hilfsstoff, der fest, flüssig oder halbflüssig sein kann, in eine geeignete Dosierungsform gebracht. Solche pharmazeutischen Zubereitungen sind ein weiterer Gegenstand der Erfindung.

Als Trägerstoff kommen dabei die üblichen, einem Fachmann bekannten organischen und anorganischen Substanzen in Frage, die je nach der vorgesehenen Applikation, also enteral, parenteral oder topisch, ausgewählt werden. Allgemeine Beispiele für derartige Substanzen sind Wasser, pflanzliche Öle, Benzylalkohole, Alkylenglykole, Polyethylenglykole, Glyzerintriacetat, Gelatine, Kohlenhydrate, beispielsweise Lactose oder Stärke, Magnesiumstearat, Talk und Vaseline. Bei der Auswahl der vorstehend erwähnten Trägersubstanzen ist dabei selbstverständlich darauf zu achten, daß diese nicht mit den erfindungsgemäßen Substanzen reagieren.

Beispiele für Verabreichungsformen bei der oralen Anwendung, die die erfindungsgemäß bevorzugte Applikationsform darstellt, sind insbesondere Tabletten, Pillen, Dragees, Kapseln, Pulver, Granulate, Sirupe, Säfte und Tropfen.

Bei der rektalen Anwendung wird insbesondere zurückgegriffen auf Suppositorien, bei der parenteralen Anwendungen werden Lösungen verwendet, vorzugsweise ölige oder wäßrige Lösungen, weiterhin finden auch Suspensionen, Emulsionen und Implantate Verwendung.

Beispiele für die topische Anwendung schließen Salben, Cremes und Puder ein.

Eine andere Möglichkeit besteht darin, die erfindungsgemäßen Verbindungen zu lyophilieren. Die Lyophilisate können dann beispielsweise zur Herstellung von Injektionspräparaten verwendet werden.

Die Zubereitungen der erfindungsgemäßen Substanzen können sterilisiert sein und/oder Hilfsstoffe wie Gleit-, Konservierungs-, Stabilisierungs- und Netzmittel, Emulgatoren, Salze zur Beeinflussung des osmotischen Drucks, Puffersubstanzen, Farb- und Geschmacksstoffe enthalten. Beispiele für solche Substanzen sind Vitamine.

Die Dosierungsmenge der erfindungsgemäßen Substanzen liegt vorzugsweise bei Mengen 1 bis 500 mg, insbesondere 5 bis 100 mg pro Dosierungseinheit. Tägliche Dosierungseinheiten sind dabei von 0,02 bis 10 mg/kg Körpergewicht. Dabei gilt jedoch, daß die individuelle Dosis von Patient zu Patient und auch für jeden einzelnen Patienten stark schwanken kann und von verschiedensten Faktoren abhängt. Solche Faktoren sind beispielsweise die Wirksamkeit der eingesetzten speziellen Verbindung, Alter, Körpergewicht, allgemeiner Gesundheitszustand, Geschlecht, während des Verabreichungszeitraums eingenommene Kost, Verabreichungszeitpunkt sowie Verabreichungsweg, Ausscheidungsgeschwindigkeit, Arzneistoffkombination sowie die Schwere der jeweiligen Erkrankung.

Die erfindungsgemäßen Verbindungen der Formel (I) werden hergestellt durch Umsetzung des entsprechenden 5-Halogenomethyl-pyrazolo[4,3-d]pyrimidins der allgemeinen Formel (II a) mit dem entsprechenden Alkylamin der Formel (III), wie dies in der nachfolgenden Gleichung (1) gezeigt ist.

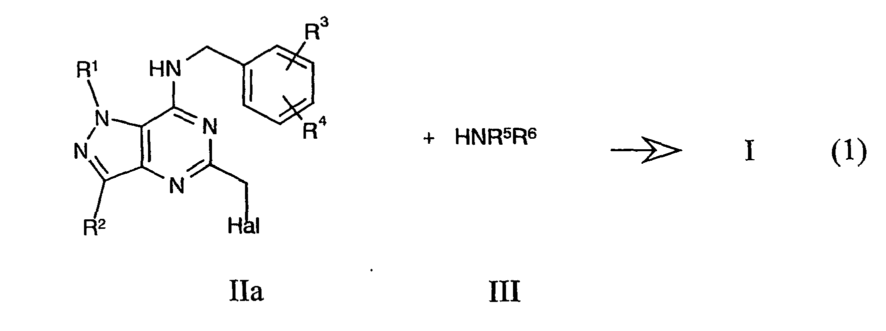

In den Formeln (II a) und (III) haben die Substituenten R¹ bis R⁶ die im Zusammenhang mit der Formel (I) angegebene Bedeutung. Hal steht für ein Halogenatom, vorzugsweise für Chlor.

Die Umsetzung nach der Gleichung (1) wird dabei bei Temperaturen von -30 bis 150°C, vorzugsweise 0 bis 120 °C, durchgeführt. Die Reaktion kann dabei in Substanz, ohne Verwendung eines Lösungsmittels, durchgeführt werden, oder unter Verwendung eines geeigneten Solvens. Als solche eignen sich generell die üblichen, einem Fachmann bekannten Lösungsmittel. Beispiele sind Kohlenwasserstoffe wie Hexan, Petrolether, Benzol, Toluol und Xylol, chlorierte Kohlenwasserstoffe wie Trichlorethylen, 1,2-Dichlorethan, Tetrachlorkohlenstoff, Chloroform und Dichlormethan, Alkohole wie Methanol, Ethanol, Isopropanol, n-Propanol, n-Butanol oder tert-Butanol, Ether wie Diethylether, Diisopropylether, Tetrahydrofuran und Dioxan, Glykolether wie Ethylenglykolmonomethyl- und monoethylether, Ethylenglykoldimethylether, Ketone wie Aceton und Butanon, Amide wie Acetamid, Dimethylacetamid, N-Methylpyrrolidon und Dimethylformamid, Nitrile wie Acetonitril, Sulfoxide wie Dimethylsulfoxid, Nitroverbindungen wie Nitromethan und Nitrobenzol, Ester wie Ethylacetat, sowie Gemische der oben genannten Lösungsmittel. Vorzugsweise werden Dimethylformaid und/oder N-Methylpyrrolidon als Solvens verwendet.

Nach beendeter Reaktion wird die erhaltene Substanz auf die übliche Weise aufgearbeitet, beispielsweise durch Extraktion mit einem organischen Lösungsmittel nach Versetzen mit Wasser und dem üblichen Isolieren der Verbindung, beispielsweise durch Abdestillieren des Lösungsmittels. Generell wird der erhaltene Rückstand zur Reinigung umkristallisiert.

Die 5-Halogenomethyl-pyrazolo[4,3-d]pyrimidine der allgemeinen Formel (II a) lassen sich aus den entsprechenden 4-Chlor-pyrazolo[4,3-d]pyrimidinen durch Umsetzung mit dem entsprechenden, gegebenenfalls substituierten Benzylamin darstellen. Die Beschreibung einer analogen Reaktion, bei der der Pyrimidinring statt mit einer Halogenomethylfunktion mit einer Gruppe X substituiert ist, wie sie in der oberen Formel (II) definiert wurde, ist in der deutschen Anmeldung Aktenzeichen 199 42 474.8 der Anmelderin beschrieben. Die dort offenbarten Reaktionsbedingungen lassen sich auf die Substanzen der vorliegenden Erfindung übertragen.

Die 4-Chlor-pyrazolopyrimidine lassen sich in literaturbekannter Weise erhalten, siehe beispielsweise Houben-Weyl, Methoden der organischen Chemie, Georg Thieme Verlag, Stuttgart.

Die Erfindung wird nun in den nachfolgenden Beispielen näher erläutert. Dabei sind die Temperaturen in °C angegeben, und die Abkürzungen haben die einem Fachmann bekannte Bedeutung. Sämtliche Produkte wurden dabei nach vollendeter Reaktion aufgearbeitet, indem Wasser zugegeben und der pH-Wert der Lösung auf Werte zwischen ca. 2 und 10, je nach erhaltenem Produkt, eingestellt wurde. Anschließend wurde mit Ethylacetat oder Dichlormethan extrahiert, die organische Phase abgetrennt und getrocknet. Danach wurde das Lösungsmittel abdestilliert und der erhaltene Rückstand durch Chromatographie an Kieselgel und/oder Kristallisation gereinigt.

### Beispiel 1

Eine Lösung von 2 g 7-(3-Chlor-4-methoxybenzylamino)-5-chlormethyl-1-methyl-3-propyl-1H-pyrazolo[4,3-d]pyrimidin in 10 ml in DMF wird mit 5 ml 3-Aminopropanol versetzt und zwei Stunden bei Raumtemperatur gerührt. Anschließend wird mit Wasser verdünnt und mit Essigester extrahiert. Das nach üblicher Aufarbeitung erhaltene ölige Produkt wird in alkoholischer HCl gelöst und mit Essigester bis zur Trübung versetzt. Die ausgefallenen Kristalle werden abgesaugt und aus Isopropanol/Essigester umkristallisiert. Man erhält 3,1 g 7-(3-Chlor-4-methoxybenzylamino)-5-(3-hydroxy-propylaminomethyl)-1-methyl-3-propyl-1H-pyrazolo[4,3-d]pyrimidin in Form seines Dihydrochlorids.
F. = 207°

### Beispiel 2

Die Umsetzung wurde durchgeführt wie in Beispiel 1 beschrieben, wobei an Stelle des 3-Aminopropanols eine äquivalente Menge von 4-Piperidinol verwendet wurde.

Nach Versetzen mit der jeweiligen Säure wurde dann das 7-(3-Chlor-4-methoxybenzylamino)-5-(4-hydroxypiperidinolmethyl)-1-methyl-3-propyl-1H-pyrazolo[4,3-d]pyrimidin in Form seinesDihydrchlorids erhalten. F.=206°.

### Beispiel 3

Die Umsetzung wurde durchgeführt wie in Beispiel 1 beschrieben, wobei an Stelle des 3-Aminopropanols eine äquivalente Menge von 2-Hydroxyethylamin verwendet wurde.

Nach Versetzen mit der jeweiligen Säure wurde dann das 7-(3-Chlor-4-methoxybenzylamino)-5-(2-hydroxyethylaminomethyl)-1-methyl-3-propyl-1H-pyrazolo[4,3-d]pyrimidin in Form seines Dihydrochlorids erhalten. F.=201°.

### Beispiel 4

Die Umsetzung wurde durchgeführt wie in Beispiel 1 beschrieben, wobei an Stelle des 3-Aminopropanols eine äquivalente Menge von N-(2Hydroxyethyl)-piperazin verwendet wurde.

Nach Versetzen mit der jeweiligen Säure wurde dann das 7-(3-Chlor-4-methoxybenzylamino)-5-(N-(2-hydroxyethyl)-piperazinomethyl)-1-methyl-3-propyl-1H-pyrazolo[4,3-d]pyrimidin in Form seines Trihydrochlorids erhalten.
F. =176°.

### Beispiel 5

Die Umsetzung wurde durchgeführt wie in Beispiel 1 beschrieben, wobei an Stelle des 3-Aminopropanols eine äquivalente Menge von 3-Methoxypropylamin verwendet wurde.

Nach Versetzen mit der jeweiligen Säure wurde dann das 7-(3-Chlor-4-methoxybenzylamino)-5-(3-methoxypropylaminomethyl)-1-methyl-3-propyl-1H-pyrazolo[4,3-d]pyrimidin in Form seines Dihydrochlorids erhalten. F.=211°.

### Beispiel 6

Die Umsetzung wurde durchgeführt wie in Beispiel 1 beschrieben, wobei an Stelle des 3-Aminopropanols eine äquivalente Menge von N,N-Dimethylethylendiamin verwendet wurde.

Nach Versetzen mit der jeweiligen Säure wurde dann das 7-(3-Chlor-4-methoxybenzylamino)-5-(N,N-dimethylaminoethylaminomethyl)-1-methyl-3-propyl-1H-pyrazolo[4,3-d]pyrimidin in Form seines Trihydrochlorids erhalten.
F.=223°.

### Analog wurden erhalten:

(3-Chloro-4-methoxy-benzyl)-(1-methyl-3-propyl-5-{[(pyridin-3-ylmethyl)-amino]-methyl}-1H-pyrazolo[4,3-d]pyrimidin-7-yl)-amin 5-{[7-(3-Chloro-4-methoxy-benzylamino)-1-methyl-3-propyl-1H-pyrazolo[4,3-d]pyrimidin-5-ylmethyl]-amino}-pentan-1-ol 3-{4-[7-(3-Chloro-4-methoxy-benzylamino)-1-methyl-3-propyl-1H-pyrazolo[4,3-d]pyrimidin-5-ylmethyl]-piperazin-1-yl}-propane-1,2-diol (3-Chloro-4-methoxy-benzyl)-[5-(4-dimethylamino-piperidin-1-ylmethyl)-1-methyl-3-propyl-1H-pyrazolo[4,3-d]pyrimidin-7-yl]-amin 2-{[7-(3-Chloro-4-methoxy-benzylamino)-1-methyl-3-propyl-1H-pyrazolo[4,3-d]pyrimidin-5-ylmethyl]-ethyl-amino}-ethanol (3-Chloro-4-methoxy-benzyl)-{1-methyl-5-[(2-morpholin-4-yl-ethylamino)-methyl]-3-propyl-1 H-pyrazolo[4,3-d]pyrimidin-7-yl}-amin (2S,3S,4S,5R)-6-{[7-(3-Chloro-4-methoxy-benzylamino)-1-methyl-3-propyl-1H-pyrazolo[4,3-d]pyrimidin-5-ylmethyl]-methyl-amino}-hexane-1,2,3,4,5-pentaol

Die nachfolgenden Beispiele betreffen pharmazeutische Zubereitungen:

### Beispiel A: Injektionsgläser

Eine Lösung von 100g eines Wirkstoffs der Formel (I) und 5 g Dinatriumhydrogenphosphat wird in 3 l zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel (I) mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel (I), 9,38 g NaH₂PO₄·2H₂O, 28,48 g Na₂HPO₄ 12H₂O und 0,1 g Benzalkoniumchlorid in 940 ml zweifach destilliertem Wasser. Man stellt auf pH 6,8 ein, füllt auf I auf und sterilisiert durch Bestrahlen. Diese Lösung kann in Form von Augentropfen verwendet werden.

### Beispiel D: Salbe

Man mischt 500 g eines Wirkstoffes der Formel (I) mit 99,5 g Vaseline unter aseptischen Bedingungen.

### Beispiel E: Tabletten

Ein Gemisch von 1 kg Wirkstoff der Formel (I), 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel (I) werden in üblicher Weise in Hartgelatinekapseln gefüllt, so daß jede Kapsel 20 mg des Wirkstoffe enthält.

### Beispiel H: Ampullen

Eine Lösung von 1 kg Wirkstoff der Formel (I) in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

### Beispiel I: Inhalations-Spray

Man löst 14 g Wirkstoff der Formel (I) in 10 I isotonischer NaCl-Lösung und füllt die Lösung in handelsübliche Sprühgefäße mit Pump-Mechanismus. Die Lösung kann in Mund oder Nase gesprüht werden. Ein Sprühstoß (etwa 0,1 ml) entspricht einer Dosis von etwa 0,14 mg.

## Patentansprüche

1. Verbindung der Formel (I) 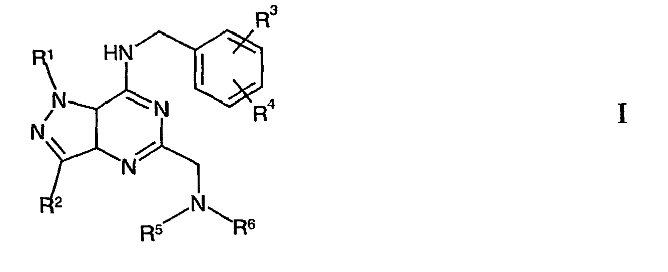 in der
R¹, R² entweder gleich oder verschieden sind und unabhängig voneinander Wasserstoff oder eine C₁-C₈-Alkylgruppe bedeuten,
R³, R⁴ entweder gleich oder verschieden sind und unabhängig voneinander Wasserstoff, eine Hydroxylgruppe, eine lineare oder verzweigte C₁-C₈-Alkylgruppe, eine C₁-C₈-Alkoxygruppe oder Halogen bedeuten, oder zusammen mit den C-Atomen, an die sie gebunden sind, einen 5-8-gliedrigen Ring bilden, der neben Kohlenstoffatomen gegebenenfalls ein oder mehrere Sauerstoffatome enthalten kann,
R⁵, R⁶ gleich oder verschieden sein können und unabhängig voneinander Wasserstoff, eine lineare oder verzweigte C₁-C₈-Alkylgruppe, die mit einer oder mehreren Hydroxyl-, C₁-C₈-Alkoxy-, Amin-, Mono(C₁-C₈-Alkyl)amin-, Di(C₁-C₈-Alkyl)amin-, Nitrilo-, N-Morpholino-, Phenyl-, Benzodioxol- oder Pyridylgruppen substituiert sein kann, oder eine C₄-C₇-Cycloalkylgruppe bedeuten, oder gemeinsam mit dem Stickstoffatom, an die sie gebunden sind, einen gesättigten heterocyclischen Ring bilden, der gegebenenfalls eines oder mehrere weitere Stickstoff- und/oder Sauerstoffatome enthält und der mit einer oder mehreren C₁-C₈-Alkyl-, Hydroxy-, C₁-C₈-Alkoxy-, C₁-C₈-Alkylol-, C₁-C₆-Oligohydroxyalkyl-, Amino-, Mono(C₁-C₈-Alkyl)-amino-, Di(C₁-C₈-Alkyl)amino-, -SO₂R⁷- oder -C(O)R⁷-Gruppen substituiert ist,
R⁷ eine C₁-C₈-Alkyl-, C₁-C₈-Fluoralkyl-, eine gegebenenfalls durch Alkyl-, Halogen- oder Nitrilgruppen substituierte Phenylgruppe oder eine Benzodioxolgruppe ist.

2. Verbindung nach Anspruch 1, **dadurch gekennzeichnet, daß** R¹, R² verschieden und ausgewählt sind aus der Gruppe bestehend aus Wasserstoff und linearen oder verzweigten (C₁-C₄)-Alkylgruppen, wobei R¹ insbesondere eine Methyl- oder Ethylgruppe und R² insbesondere eine Propyl- oder Butylgruppe ist,
R³ und R⁴ gleich oder verschieden sind, sich in den Positionen 3 und 4 des Phenylrings befinden, unabhängig voneinander Wasserstoff, eine lineare oder verzweigte (C₁-C₆)-Alkylgruppe, eine lineare oder verzweigte (C₁-C₆)-Alkoxygruppe oder Halogen bedeuten, oder zusammen eine Propylen-, Butylen-, Pentylen-, Ethylenoxy-, Methylenoxy- oder Ethylendioxygruppe bilden,
R⁵ und R⁶ gleich oder verschieden sind und unabhängig voneinander Wasserstoff oder eine C₁-C₆-Alkylgruppe, die unsubstituiert ist oder mit einer oder mehreren Hydroxy-, Methoxy-, Ethoxy-, Nitril-, Methylamin-, Ethylamin-, Dimethylamin-, Diethylamin-, Pyridyl-, Benzodioxol- oder N-Morpholinogruppen substituiert ist, oder eine Cyclopentyl- oder Cyclohexylgruppe bedeuten, oder R⁵, R⁶ zusammen mit dem Stickstoffatom, an das sie gebunden sind, einen Piperidinyl- oder Piperazinylring, der gegebenenfalls mit einer oder mehreren Hydroxy-, Hydroxycarbonyl-, C₁-C₂-Alkylamin-, -SO₂-R⁷ oder -C(O)-R⁷-Gruppen substituiert ist, bilden, wobei R⁷ eine C₁-C₃-Alkyl-, eine C₁-C₃-Fluoralkyl-, eine durch eine oder mehrere Alkyl- oder Nitrilgruppen substituierte Phenylgruppe oder eine Benzodioxolgruppe ist.

3. 7-(3-Chlor-4-methoxybenzylamino)-5-(3-hydroxypropylaminomethyl)-1-methyl-3-propyl-1H-pyrazolo[4,3-d]pyrimidin, 7-(3-Chlor-4-methoxybenzylamino)-5-(4-hydroxypiperidinomethyl)-1-methyl-3-propyl-1H-pyrazolo[4,3-d]pyrimidin, 7-(3-Chlor-4-methoxybenzylamino)-5-(2-hydroxyethylaminomethyl)-1-methyl-3-propyl-1H-pyrazolo[4,3-d]pyrimidin, 7-(3-Chlor-4-methoxybenzylamino)-5-(N-(2-hydroxyethyl)-piperazinomethyl)-1-methyl-3-propyl-1H-pyrazolo[4,3-d]pyrimidin, 7-(3-Chlor-4-methoxybenzylamino)-5-(3-methoxypropylaminomethyl)-1-methyl-3-propyl-1H-pyrazolo[4,3-d]pyrimidin, 7-(3-Chlor-4-methoxybenzylamino)-5-(N,N-dimethylaminoethylaminomethyl)-1-methyl-3-propyl-1H-pyrazolo[4,3-d]pyrimidin, (3-Chloro-4-methoxy-benzyl)-(1-methyl-3-propyl-5-{[(pyridin-3-ylmethyl)-amino]-methyl}-1H-pyrazolo[4,3-d]pyrimidin-7-yl)-amin 5-{[7-(3-Chloro-4-methoxy-benzylamino)-1-methyl-3-propyl-1H-pyrazolo[4,3-d]pyrimidin-5-ylmethyl]-amino}-pentan-1-ol 3-{4-[7-(3-Chloro-4-methoxy-benzylamino)-1-methyl-3-propyl-1H-pyrazolo[4,3-d]pyrimidin-5-ylmethyl]-piperazin-1-yl}-propane-1,2-diol (3-Chloro-4-methoxy-benzyl)-[5-(4-dimethylamino-piperidin-1-ylmethyl)-1-methyl-3-propyl-1H-pyrazolo[4,3-d]pyrimidin-7-yl]-amin 2-{[7-(3-Chloro-4-methoxy-benzylamino)-1-methyl-3-propyl-1H-pyrazolo[4,3-d]pyrimidin-5-ylmethyl]-ethyl-amino}-ethanol (3-Chloro-4-methoxy-benzyl)-{1-methyl-5-[(2-morpholin-4-yl-ethylamino)-methyl]-3-propyl-1H-pyrazolo[4,3-d]pyrimidin-7-yl}-amin (2S,3S,4S,5R)-6-{[7-(3-Chloro-4-methoxy-benzylamino)-1-methyl-3-propyl-1H-pyrazolo[4,3-d]pyrimidin-5-ylmethyl]-methyl-amino}-hexane-1,2,3,4,5-pentaol
oder eines ihrer physiologisch unbedenklichen Salze.

4. Arzneimittel, enthaltend eine Verbindung nach einem der Ansprüche 1 bis 3 oder eines ihrer physiologisch unbedenklichen Salze.

5. Arzneimittel, enthaltend eine Verbindung aus der Gruppe bestehend aus 7-(3-Chlor-4-methoxybenzylamino)-5-(3-hydroxypropylaminomethyl)-1-methyl-3-propyl-1H-pyrazolo [4,3-d]pyrimidin, 7-(3-Chlor-4-methoxybenzylamino)-5-(4-hydroxypiperidinolmethyl)-1-methyl-3-propyl-1H-pyrazolo[4,3-d]pyrimidin, 7-(3-Chlor-4-methoxybenzylamino)-5-(2-hydroxyethylaminomethyl)-1-methyl-3-propyl-1H-pyrazolo[4,3-d]pyrimidin, 7-(3-Chlor-4-methoxybenzylamino)-5-(N-(2-hydroxyethyl)-piperazinomethyl)-1-methyl-3-propyl-1H-pyrazolo[4,3-d]pyrimidin, 7-(3-Chlor-4-methoxybenzylamino)-5-(3-methoxypropylaminomethyl)-1-methyl-3-propyl-1H-pyrazolo[4,3-d]pyrimidin, 7-(3-Chlor-4-methoxybenzylamino)-5-(N,N-dimethylaminoethylaminomethyl)-1-methyl-3-propyl-1H-pyrazolo[4,3-d]pyrimidin, (3-Chloro-4-methoxy-benzyl)-(1-methyl-3-propyl-5-{[(pyridin-3-ylmethyl)-amino]-methyl}-1H-pyrazolo[4,3-d]pyrimidin-7-yl)-amin 5-{[7-(3-Chloro-4-methoxy-benzylamino)-1-methyl-3-propyl-1H-pyrazolo[4,3-d]pyrimidin-5-ylmethyl]-amino}-pentan-1-ol 3-{4-[7-(3-Chloro-4-methoxy-benzylamino)-1-methyl-3-propyl-1H-pyrazolo[4,3-d]pyrimidin-5-ylmethyl]-piperazin-1-yl}-propane-1,2-diol (3-Chloro-4-methoxy-benzyl)-[5-(4-dimethylamino-piperidin-1-ylmethyl)-1-methyl-3-propyl-1H-pyrazolo[4,3-d]pyrimidin-7-yl]-amin 2-{[7-(3-Chloro-4-methoxy-benzylamino)-1-methyl-3-propyl-1H-pyrazolo[4,3-d]pyrimidin-5-ylmethyl]-ethyl-amino}ethanol (3-Chloro-4-methoxy-benzyl)-{1-methyl-5-[(2-morpholin-4-yl-ethylamino)-methyl]-3-propyl-1H-pyrazolo[4,3-d]pyrimidin-7-yl}-amin (2S,3S,4S,5R)-6-{[7-(3-Chloro-4-methoxy-benzylamino)-1-methyl-3-propyl-1H-pyrazolo[4,3-d]pyrimidin-5-ylmethyl]-methyl-amino}-hexane-1,2,3,4,5-pentaol
oder eines ihrer physiologisch unbedenklichen Salze.

6. Verwendung einer Verbindung nach einem der Ansprüche 1 bis 3 oder einer ihrer physiologisch unbedenklichen Salze zur Herstellung eines Arzneimittels zur c-GMP-Phosphodiesterasehemmung.

7. Verwendung einer Verbindung nach einem der Ansprüche 1 bis 3 oder eines ihrer physiologisch unbedenklichen Salze zur Herstellung eines Arzneimittels gegen Herz-Kreislaufkrankheiten.

8. Verwendung einer Verbindung nach einem der Ansprüche 1 bis 3 oder eines ihrer physiologisch unbedenklichen Salze zur Herstellung eines Arzneimittels gegen Potenzstörungen.

9. Verwendung nach einem der Ansprüche 6 bis 8, **dadurch gekennzeichnet, daß** eine Verbindung verwendet wird, die ausgewählt ist aus der Gruppe bestehend aus 7-(3-Chlor-4-methoxybenzylamino)-5-(3-hydroxypropylaminomethyl)-1-methyl-3-propyl-1H-pyrazolo[4,3-d]pyrimidin, 7-(3-Chlor-4-methoxybenzylamino)-5-(4-hydroxypiperidinolmethyl)-1-methyl-3-propyl-1H-pyrazolo[4,3-d]pyrimidin, 7-(3-Chlor-4-methoxybenzylamino)-5-(2-hydroxyethylaminomethyl)-1-methyl-3-propyl-1H-pyrazolo[4,3-d]pyrimidin, 7-(3-Chlor-4-methoxybenzylamino)-5-(N-(2-hydroxyethyl)-piperazinomethyl)-1-methyl-3-propyl-1H-pyrazolo[4,3-d]pyrimidin, 7-(3-Chlor-4-methoxybenzylamino)-5-(3-methoxypropylaminomethyl)-1-methyl-3-propyl-1H-pyrazolo[4,3-d]pyrimidin, 7-(3-Chlor-4-methoxybenzylamino)-5-(N,N-dimethylaminoethylaminomethyl)-1-methyl-3-propyl-1H-pyrazolo[4,3-d]pyrimidin, (3-Chloro-4-methoxy-benzyl)-(1-methyl-3-propyl-5-{[(pyridin-3-ylmethyl)-amino]-methyl}-1H-pyrazolo[4,3-d]pyrimidin-7-yl)-amin 5-{[7-(3-Chloro-4-methoxy-benzylamino)-1-methyl-3-propyl-1H-pyrazolo[4,3-d]pyrimidin-5-ylmethyl]-amino}-pentan-1-ol 3-{4-[7-(3-Chloro-4-methoxy-benzylamino)-1-methyl-3-propyl-1H-pyrazolo[4,3-d]pyrimidin-5-ylmethyl]-piperazin-1-yl}-propane-1,2-diol (3-Chloro-4-methoxy-benzyl)-[5-(4-dimethylamino-piperidin-1-ylmethyl)-1-methyl-3-propyl-1H-pyrazolo[4,3-d]pyrimidin-7-yl]-amin 2-{[7-(3-Chloro-4-methoxy-benzylamino)-1-methyl-3-propyl-1H-pyrazolo[4,3-d]pyrimidin-5-ylmethyl]-ethyl-amino}-ethanol (3-Chloro-4-methoxy-benzyl)-{1-methyl-5-[(2-morpholin-4-yl-ethylamino)-methyl]-3-propyl-1H-pyrazolo[4,3-d]pyrimidin-7-yl}-amin (2S,3S,4S,5R)-6-{[7-(3-Chloro-4-methoxy-benzylamino)-1-methyl-3-propyl-1H-pyrazolo[4,3-d]pyrimidin-5-ylmethyl]-methyl-amino}-hexane-1,2,3,4,5-pentaol
oder einem ihrer physiologisch unbedenklichen Salze.

10. Verfahren zur Herstellung einer Verbindung nach einem der Ansprüche 1 bis 3, **dadurch gekennzeichnet, daß** ein 5-Halogenomethylpyrazolo[4,3-d]pyrimidin der allgemeinen Formel (IIa) 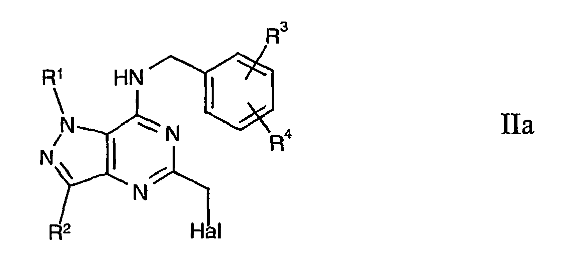 in dem die Substituenten R¹ bis R⁴ die in Anspruch 1 angegebene Bedeutung besitzen, mit einem Alkylamin der allgemeinen Formel (III)
HNR⁵R⁶ III
in dem die Substituenten R⁵ und R⁶, die in Anspruch 1 angegebenen Bedeutung haben, umsetzt und die erhaltene Substanz gegebenenfalls reinigt.

11. Verfahren zur Herstellung eines Arzneimittels, **dadurch gekennzeichnet, daß** man eine Verbindung nach einem der Ansprüche 1 bis 3 oder eines ihre physiologisch unbedenklichen Salze mit mindestens einem geeigneten Hilfs- oder Trägerstoff in eine geeignete Dosierungsform bringt.

## Claims

1. Compound of the formula (I) 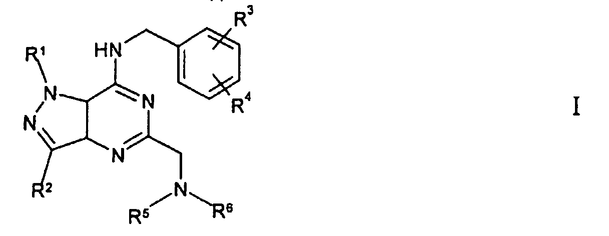 in which
R¹, R² are either identical or different and, independently of one another, denote hydrogen or a C₁-C₈-alkyl group,
R³, R⁴ are either identical or different and, independently of one another, denote hydrogen, a hydroxyl group, a linear or branched C₁-C₈-alkyl group, a C₁-C₈-alkoxy group or halogen, or, together with the C atoms to which they are bonded, form a 5- to 8-membered ring which, besides carbon atoms, may optionally contain one or more oxygen atoms,
R⁵, R⁶ may be identical or different and, independently of one another, denote hydrogen, a linear or branched C₁-C₈-alkyl group, which may be substituted by one or more hydroxyl, C₁-C₈-alkoxy, amine, mono(C₁-C₈-alkyl)amine, di(C₁-C₈-alkyl)amine, nitrilo, N-morpholino, phenyl, benzodioxole or pyridyl groups, or a C₄-C₇-cycloalkyl group, or, together with the nitrogen atom to which they are bonded, form a saturated heterocyclic ring, which optionally contains one or more further nitrogen and/or oxygen atoms and which is substituted by one or more C₁-C₈-alkyl, hydroxyl, C₁-C₈-alkoxy, C₁-C₈-alkylol, C₁-C₆-oligohydroxyalkyl, amino, mono(C₁-C₈-alkyl)amino, di(C₁-C₈-alkyl)amino, -SO₂R⁷ or -C(O)R⁷ groups,
R⁷ is a C₁-C₈-alkyl group, a C₁-C₈-fluoroalkyl group, a phenyl group, which is optionally substituted by alkyl, halogen or nitrile groups, or a benzodioxole group.

2. Compound according to Claim 1, **characterised in that** R¹, R² are different and are selected from the group consisting of hydrogen and linear or branched (C₁-C₄)alkyl groups, where R¹ is in particular a methyl or ethyl group and R² is in particular a propyl or butyl group,
R³ and R⁴ are identical or different, are located in positions 3 and 4 of the phenyl ring, denote, independently of one another, hydrogen, a linear or branched (C₁-C₆)alkyl group, a linear or branched (C₁-C₆)alkoxy group or halogen, or together form a propylene, butylene, pentylene, ethylenoxy, methylenoxy or ethylenedioxy group,
R⁵ and R⁶ are identical or different and, independently of one another, denote hydrogen or a C₁-C₆-alkyl group, which is unsubstituted or substituted by one or more hydroxyl, methoxy, ethoxy, nitrile, methylamine, ethylamine, dimethylamine, diethylamine, pyridyl, benzodioxole or N-morpholino groups, or a cyclopentyl or cyclohexyl group, or R⁵, R⁶, together with the nitrogen atom to which they are bonded, form a piperidinyl or piperazinyl ring, which is optionally substituted by one or more hydroxyl, hydroxycarbonyl, C₁-C₂-alkylamine, -SO₂-R⁷ or -C(O)-R⁷ groups, where R⁷ is a C₁-C₃-alkyl group, a C₁-C₃-fluoroalkyl group, a phenyl group which is substituted by one or more alkyl or nitrile groups, or a benzodioxole group.

3. 7-(3-Chloro-4-methoxybenzylamino)-5-(3-hydroxypropylaminomethyl)-1-methyl-3-propyl-1H-pyrazolo[4,3-d]pyrimidine, 7-(3-chloro-4-methoxybenzylamino)-5-(4-hydroxypiperidinomethyl)-1-methyl-3-propyl-1H-pyrazolo[4,3-d]pyrimidine, 7-(3-chloro-4-methoxybenzylamino)-5-(2-hydroxyethylaminomethyl)-1-methyl-3-propyl-1H-pyrazolo[4,3-d]pyrimidine, 7-(3-chloro-4-methoxybenzylamino)-5-(N-(2-hydroxyethyl)piperazinomethyl)-1-methyl-3-propyl-1H-pyrazolo[4,3-d]-pyrimidine, 7-(3-chloro-4-methoxybenzylamino)-5-(3-methoxypropylaminomethyl)-1-methyl-3-propyl-1H-pyrazolo[4,3-d]pyrimidine, 7-(3-chloro-4-methoxybenzylamino)-5-(N,N-dimethylaminoethylaminomethyl)-1-methyl-3-propyl-1H-pyrazolo[4,3-d]pyrimidine, (3-chloro-4-methoxybenzyl)(1-methyl-3-propyl-5-{[(pyridin-3-ylmethyl)amino]-methyl}-1H-pyrazolo[4,3-d]pyrimidin-7-yl)amine, 5-{[7-(3-chloro-4-methoxybenzylamino)-1-methyl-3-propyl-1H-pyrazolo[4,3-d]pyrimidin-5-ylmethyl]amino}pentan-1-ol, 3-{4-[7-(3-chloro-4-methoxybenzylamino)-1-methyl-3-propyl-1H-pyrazolo[4,3-d]pyrimidin-5-ylmethyl]piperazin-1-yl]}propane-1,2-diol, (3-chloro-4-methoxybenzyl)[5-(4-dimethylaminopiperidin-1-ylmethyl)-1-methyl-3-propyl-1H-pyrazolo[4,3-d]-pyrimidin-7-yl]amine, 2-{[7-(3-chloro-4-methoxybenzylamino)-1-methyl-3-propyl-1H-pyrazolo[4,3-d]pyrimidin-5-ylmethyl]ethylamino}ethanol, (3-chloro-4-methoxybenzyl){1-methyl-5-[(2-morpholin-4-ylethylamino)-methyl]-3-propyl-1H-pyrazolo[4,3-d]pyrimidin-7-yl}amine, (2S,3S,4S,5R)-6-{[7-(3-chloro-4-methoxybenzylamino)-1-methyl-3-propyl-1H-pyrazolo[4,3-d]pyrimidin-5-ylmethyl]methylamino}hexane-1,2,3,4,5-pentaol
or one of the physiologically acceptable salts thereof.

4. Medicament comprising a compound according to one of Claims 1 to 3 or one of the physiologically acceptable salts thereof.

5. Medicament comprising a compound from the group consisting of 7-(3-chloro-4-methoxybenzylamino)-5-(3-hydroxypropylaminomethyl)-1-methyl-3-propyl-1H-pyrazolo[4,3-d]pyrimidine, 7-(3-chloro-4-methoxybenzylamino)-5-(4-hydroxypiperidinomethyl)-1-methyl-3-propyl-1H-pyrazolo[4,3-d]pyrimidine, 7-(3-chloro-4-methoxybenzylamino)-5-(2-hydroxyethylaminomethyl)-1-methyl-3-propyl-1H-pyrazolo[4,3-d]-pyrimidine, 7-(3-chloro-4-methoxybenzylamino)-5-(N-(2-hydroxyethyl)-piperazinomethyl)-1-methyl-3-propyl-1H-pyrazolo[4,3-d]pyrimidine, 7-(3-chloro-4-methoxybenzylamino)-5-(3-methoxypropylaminomethyl)-1-methyl-3-propyl-1H-pyrazolo[4,3-d]pyrimidine, 7-(3-chloro-4-methoxybenzylamino)-5-(N,N-dimethylaminoethylaminomethyl)-1-methyl-3-propyl-1H-pyrazolo[4,3-d]pyrimidine, (3-chloro-4-methoxybenzyl)(1-methyl-3-propyl-5-{[(pyridin-3-ylmethyl)amino]methyl}-1H-pyrazolo[4,3-d]pyrimidin-7-yl)amine, 5-{[7-(3-chloro-4-methoxybenzylamino)-1-methyl-3-propyl-1H-pyrazolo[4,3-d]pyrimidin-5-ylmethyl]-amino}pentan-1-ol, 3-{4-[7-(3-chloro-4-methoxybenzylamino)-1-methyl-3-propyl-1H-pyrazolo[4,3-d]pyrimidin-5-ylmethyl]piperazin-1-yl]}-propane-1,2-diol, (3-chloro-4-methoxybenzyl)[5-(4-dimethylaminopiperidin-1-ylmethyl)-1-methyl-3-propyl-1H-pyrazolo[4,3-d]pyrimidin-7-yl]amine, 2-{[7-(3-chloro-4-methoxybenzylamino)-1-methyl-3-propyl-1H-pyrazolo[4,3-d]pyrimidin-5-ylmethyl]ethylamino}ethanol, (3-chloro-4-methoxybenzyl){1-methyl-5-[(2-morpholin-4-ylethylamino)methyl]-3-propyl-1H-pyrazolo[4,3-d]pyrimidin-7-yl}amine, (2S,3S,4S,5R)-6-{[7-(3-chloro-4-methoxybenzylamino)-1-methyl-3-propyl-1H-pyrazolo[4,3-d]-pyrimidin-5-ylmethyl]methylamino}hexane-1,2,3,4,5-pentaol or one of the physiologically acceptable salts thereof.

6. Use of a compound according to one of Claims 1 to 3 or one of the physiologically acceptable salts thereof for the preparation of a medicament for c-GMP phosphodiesterase inhibition.

7. Use of a compound according to one of Claims 1 to 3 or one of the physiologically acceptable salts thereof for the preparation of a medicament for cardiovascular diseases.

8. Use of a compound according to one of Claims 1 to 3 or one of the physiologically acceptable salts thereof for the preparation of a medicament for impotence.

9. Use according to one of Claims 6 to 8, **characterised in that** a compound is used which is selected from the group consisting of 7-(3-chloro-4-methoxybenzylamino)-5-(3-hydroxypropylaminomethyl)-1-methyl-3-propyl-1H-pyrazolo[4,3-d]pyrimidine, 7-(3-chloro-4-methoxybenzylamino)-5-(4-hydroxypiperidinomethyl)-1-methyl-3-propyl-1H-pyrazolo[4,3-d]pyrimidine, 7-(3-chloro-4-methoxybenzylamino)-5-(2-hydroxyethylaminomethyl)-1-methyl-3-propyl-1H-pyrazolo[4,3-d]pyrimidine, 7-(3-chloro-4-methoxybenzylamino)-5-(N-(2-hydroxyethyl)piperazinomethyl)-1-methyl-3-propyl-1H-pyrazolo-[4,3-d]pyrimidine, 7-(3-chloro-4-methoxybenzylamino)-5-(3-methoxypropylaminomethyl)-1-methyl-3-propyl-1H-pyrazolo[4,3-d]pyrimidine, 7-(3-chloro-4-methoxybenzylamino)-5-(N,N-dimethylaminoethylaminomethyl)-1-methyl-3-propyl-1H-pyrazolo[4,3-d]pyrimidine, (3-chloro-4-methoxybenzyl)(1-methyl-3-propyl-5-{[(pyridin-3-yl-methyl)amino]methyl}-1H-pyrazolo[4,3-d]pyrimidin-7-yl)amine, 5-{[7-(3-chloro-4-methoxybenzylamino)-1-methyl-3-propyl-1H-pyrazolo-[4,3-d]pyrimidin-5-ylmethyl]amino}pentan-1-ol, 3-{4-[7-(3-chloro-4-methoxybenzylamino)-1-methyl-3-propyl-1H-pyrazolo[4,3-d]pyrimidin-5-ylmethyl]piperazin-1-yl]}propane-1,2-diol, (3-chloro-4-methoxybenzyl)[5-(4-dimethylaminopiperidin-1-ylmethyl)-1-methyl-3-propyl-1H-pyrazolo[4,3-d]pyrimidin-7-yl]amine, 2-{[7-(3-chloro-4-methoxybenzylamino)-1-methyl-3-propyl-1H-pyrazolo[4,3-d]pyrimidin-5-yl-methyl]ethylamino}ethanol, (3-chloro-4-methoxybenzyl){1-methyl-5-[(2-morpholin-4-ylethylamino)methyl]-3-propyl-1H-pyrazolo[4,3-d]-pyrimidin-7-yl}amine, (2S,3S,4S,5R)-6-{[7-(3-chloro-4-methoxy-benzylamino)-1-methyl-3-propyl-1H-pyrazolo[4,3-d]pyrimidin-5-yl-methyl]methylamino}hexane-1,2,3,4,5-pentaol
or one of the physiologically acceptable salts thereof.

10. Process for the preparation of a compound according to one of Claims 1 to 3, **characterised in that** a 5-halomethylpyrazolo[4,3-d]-pyrimidine of the general formula (IIa) 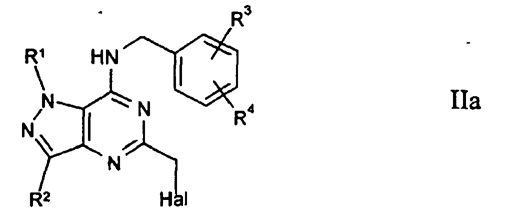 in which the substituents R¹ to R⁴ have the meaning indicated in Claim 1, is reacted with an alkylamine of the general formula (III)
HNR⁵R⁶ III
in which the substituents R⁵ and R⁶ have the meaning indicated in Claim 1, and the substance obtained is optionally purified.

11. Process for the preparation of a medicament, **characterised in that** a compound according to one of Claims 1 to 3 or one of the physiologically acceptable salts thereof is brought into a suitable dosage form with at least one suitable adjuvant or excipient.

## Revendications

1. Composé de formule (I) 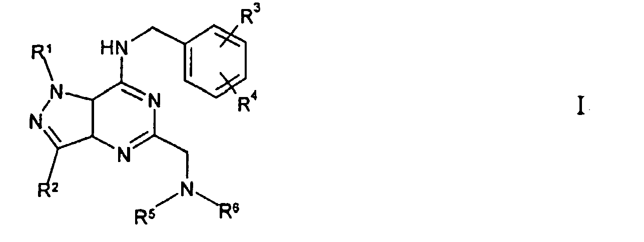 dans laquelle
R¹, R² sont identiques ou bien différents et, indépendamment l'un de l'autre, désignent hydrogène ou un groupement C₁-C₈-alkyle,
R³, R⁴ sont identiques ou bien différents et, indépendamment l'un de l'autre, désignent hydrogène, un groupement hydroxyle, un groupement C₁-C₈-alkyle linéaire ou ramifié, un groupement C₁-C₈-alcoxy ou halogène, ou, conjointement avec les atomes de C auxquels ils sont liés, forment un cycle de 5 à 8 chaînons qui, outre les atomes de carbone, peut éventuellement contenir un ou plusieurs atomes d'oxygène,
R⁵, R⁶ peuvent être identiques ou différents et, indépendamment l'un de l'autre, désignent hydrogène, un groupement C₁-C₈-alkyle linéaire ou ramifié, pouvant être substitué par un ou plusieurs groupements hydroxyle, C₁-C₈-alcoxy, amine, mono(C₁-C₈-alkyl)-amine, di(C₁-C₈-alkyl)amine, nitrilo, N-morpholino, phényle, benzodioxole ou pyridyle, ou un groupement C₄-C₇-cycloalkyle, ou, conjointement avec l'atome d'azote auquel ils sont liés, forment un cycle hétérocyclique saturé, qui contient éventuellement un ou plusieurs autres atomes d'azote et/ou d'oxygène et qui est substitué par un ou plusieurs groupements C₁-C₈-alkyle, hydroxyle, C₁-C₈-alcoxy, C₁-C₈-alkylol, C₁-C₆-oligohydroxyalkyle, amino, mono(C₁-C₈-alkyl)amino, di(C₁-C₈-alkyl)amino, -SO₂R⁷ ou -C(O)R⁷,
R⁷ est un groupement C₁-C₈-alkyle, un groupement C₁-C₈-fluoroalkyle, un groupement phényle, qui est éventuellement substitué par des groupements alkyle, halogène ou nitrile, ou un groupement benzodioxole.

2. Composé selon la revendication 1, **caractérisé en ce que** R¹, R² sont différents et sont choisis dans le groupe constitué par hydrogène et des groupements (C₁-C₄)alkyle linéaires ou ramifiés, où R¹ est en particulier un groupement méthyle ou éthyle et R² est en particulier un groupement propyle ou butyle,
R³ et R⁴ sont identiques ou différents, sont situés en positions 3 et 4 du cycle phényle, et désignent, indépendamment l'un de l'autre, hydrogène, un groupement (C₁-C₆)alkyle linéaire ou ramifié, un groupement (C₁-C₆)alcoxy linéaire ou ramifié ou halogène, ou forment ensemble un groupement propylène, butylène, pentylène, éthylèneoxy, méthylèneoxy ou éthylènedioxy,
R⁵ et R⁶ sont identiques ou différents et, indépendamment l'un de l'autre, désignent hydrogène ou un groupement C₁-C₆-alkyle, qui est non substitué ou substitué par un ou plusieurs groupements hydroxyle, méthoxy, éthoxy, nitrile, méthylamine, éthylamine, diméthylamine, diéthylamine, pyridyle, benzodioxole ou N-morpholino, ou un groupement cyclopentyle ou cyclohexyle, ou R⁵, R⁶, conjointement avec l'atome d'azote auquel ils sont liés, forment un cycle pipéridinyle ou pipérazinyle, qui est éventuellement substitué par un ou plusieurs groupements hydroxyle, hydroxycarbonyle, C₁-C₂-alkylamine, -SO₂-R⁷ ou -C(O)-R⁷, où R⁷ est un groupement C₁-C₃-alkyle, un groupement C₁-C₃-fluoroalkyle, un groupement phényle qui est substitué par un ou plusieurs groupements alkyle ou nitrile, ou un groupement benzodioxole.

3. 7-(3-Chloro-4-méthoxybenzylamino)-5-(3-hydroxypropylaminométhyl)-1-méthyl-3-propyl-1H-pyrazolo[4,3-d]pyrimidine, 7-(3-chloro-4-méthoxybenzylamino)-5-(4-hydroxypipéridinométhyl)-1-méthyl-3-propyl-1H-pyrazolo[4,3-d]pyrimidine, 7-(3-chloro-4-méthoxybenzylamino)-5-(2-hydroxyéthylaminométhyl)-1-méthyl-3-propyl-1H-pyrazolo[4,3-d]pyrimidine, 7-(3-chloro-4-méthoxybenzylamino)-5-(N-(2-hydroxyéthyl)pipérazinométhyl)-1-méthyl-3-propyl-1H-pyrazolo[4,3-d]-pyrimidine, 7-(3-chloro-4-méthoxybenzylamino)-5-(3-méthoxypropylaminométhyl)-1-méthyl-3-propyl-1H-pyrazolo[4,3-d]pyrimidine, 7-(3-chloro-4-méthoxybenzylamino)-5-(N,N-diméthylaminoéthylaminométhyl)-1-méthyl-3-propyl-1H-pyrazolo[4,3-d]pyrimidine, (3-chloro-4-méthoxybenzyl)(1-méthyl-3-propyl-5-{[(pyridin-3-ylméthyl)amino]-méthyl}-1H-pyrazolo[4,3-d]pyrimidin-7-yl)amine, 5-{[7-(3-chloro-4-méthoxybenzylamino)-1-méthyl-3-propyl-1H-pyrazolo[4,3-d]pyrimidin-5-ylméthyl]amino}pentan-1-ol, 3-{4-[7-(3-chloro-4-méthoxybenzylamino)-1-méthyl-3-propyl-1H-pyrazolo[4,3-d]pyrimidin-5-ylméthyl]pipérazin-1-yl]}propane-1,2-diol, (3-chloro-4-méthoxybenzyl)[5-(4-diméthylaminopipéridin-1-ylméthyl)-1-méthyl-3-propyl-1H-pyrazolo[4,3-d]-pyrimidin-7-yl]amine, 2-{[7-(3-chloro-4-méthoxybenzylamino)-1-méthyl-3-propyl-1H-pyrazolo[4,3-d]pyrimidin-5-ylméthyl]éthylamino}éthanol, (3-chloro-4-méthoxybenzyl){1-méthyl-5-[(2-morpholin-4-yléthylamino)-méthyl]-3-propyl-1H-pyrazolo[4,3-d]pyrimidin-7-yl}amine, (2S,3S,4S,5R)-6-{[7-(3-chloro-4-méthoxybenzylamino)-1-méthyl-3-propyl-1H-pyrazolo[4,3-d]pyrimidin-5-ylméthyl]méthylamino}hexane-1,2,3,4,5-pentaol
ou l'un des sels physiologiquement acceptables de ceux-ci.

4. Médicament comprenant un composé selon l'une des revendications 1 à 3 ou l'un des sels physiologiquement acceptables de celui-ci.

5. Médicament comprenant un composé issu du groupe constitué par la 7-(3-chloro-4-méthoxybenzylamino)-5-(3-hydroxypropylaminométhyl)-1-méthyl-3-propyl-1H-pyrazolo[4,3-d]pyrimidine, la 7-(3-chloro-4-méthoxybenzylamino)-5-(4-hydroxypipéridinométhyl)-1-méthyl-3-propyl-1H-pyrazolo[4,3-d]pyrimidine, la 7-(3-chloro-4-méthoxybenzylamino)-5-(2-hydroxyéthylaminométhyl)-1-méthyl-3-propyl-1H-pyrazolo[4,3-d]pyrimidine, la 7-(3-chloro-4-méthoxybenzylamino)-5-(N-(2-hydroxyéthyl)pipérazinométhyl)-1-méthyl-3-propyl-1H-pyrazolo[4,3-d]-pyrimidine, la 7-(3-chloro-4-méthoxybenzylamino)-5-(3-méthoxypropylaminométhyl)-1-méthyl-3-propyl-1H-pyrazolo[4,3-d]pyrimidine, la 7-(3-chloro-4-méthoxybenzylamino)-5-(N,N-diméthylaminoéthylaminométhyl)-1-méthyl-3-propyl-1H-pyrazolo[4,3-d]pyrimidine, la (3-chloro-4-méthoxybenzyl)(1-méthyl-3-propyl-5-{[(pyridin-3-ylméthyl)amino]-méthyl}-1H-pyrazolo[4,3-d]pyrimidin-7-yl)amine, le 5-{[7-(3-chloro-4-méthoxybenzylamino)-1-méthyl-3-propyl-1H-pyrazolo[4,3-d]pyrimidin-5-ylméthyl]amino}pentan-1-ol, le 3-{4-[7-(3-chloro-4-méthoxybenzylamino)-1-méthyl-3-propyl-1H-pyrazolo[4,3-d]pyrimidin-5-ylméthyl]-pipérazin-1-yl]}propane-1,2-diol, la (3-chloro-4-méthoxybenzyl)[5-(4-diméthylaminopipéridin-1-ylméthyl)-1-méthyl-3-propyl-1H-pyrazolo-[4,3-d]pyrimidin-7-yl]amine, le 2-{[7-(3-chloro-4-méthoxybenzylamino)-1-méthyl-3-propyl-1H-pyrazolo[4,3-d]pyrimidin-5-ylméthyl]éthylamino}-éthanol, la (3-chloro-4-méthoxybenzyl){1-méthyl-5-[(2-morpholin-4-yl-éthylamino)méthyl]-3-propyl-1H-pyrazolo[4,3-d]pyrimidin-7-yl}amine, le (2S,3S,4S,5R)-6-{[7-(3-chloro-4-méthoxybenzylamino)-1-méthyl-3-propyl-1H-pyrazolo[4,3-d]pyrimidin-5-ylméthyl]méthylamino}hexane-1,2,3,4,5-pentaol
ou l'un des sels physiologiquement acceptables de celui-ci.

6. Utilisation d'un composé selon l'une des revendications 1 à 3 ou de l'un des sels physiologiquement acceptables de celui-ci, pour la préparation d'un médicament destiné à l'inhibition de la c-GMP phosphodiestérase.

7. Utilisation d'un composé selon l'une des revendications 1 à 3 ou de l'un des sels physiologiquement acceptables de celui-ci, pour la préparation d'un médicament destiné aux maladies cardiovasculaires.

8. Utilisation d'un composé selon l'une des revendications 1 à 3 ou de l'un des sels physiologiquement acceptables de celui-ci, pour la préparation d'un médicament destiné à l'impuissance.

9. Utilisation selon l'une des revendications 6 à 8, **caractérisé en ce que** l'on utilise un composé choisi dans le groupe constitué par la 7-(3-chloro-4-méthoxybenzylamino)-5-(3-hydroxypropylaminométhyl)-1-méthyl-3-propyl-1H-pyrazolo[4,3-d]pyrimidine, la 7-(3-chloro-4-méthoxybenzylamino)-5-(4-hydroxypipéridinométhyl)-1-méthyl-3-propyl-1H-pyrazolo[4,3-d]pyrimidine, la 7-(3-chloro-4-méthoxybenzylamino)-5-(2-hydroxyéthylaminométhyl)-1-méthyl-3-propyl-1H-pyrazolo[4,3-d]pyrimidine, la 7-(3-chloro-4-méthoxybenzylamino)-5-(N-(2-hydroxyéthyl)pipérazinométhyl)-1-méthyl-3-propyl-1H-pyrazolo[4,3-d]pyrimidine, la 7-(3-chloro-4-méthoxybenzylamino)-5-(3-méthoxypropylaminométhyl)-1-méthyl-3-propyl-1H-pyrazolo-[4,3-d]pyrimidine, la 7-(3-chloro-4-méthoxybenzylamino)-5-(N,N-diméthylaminoéthylaminométhyl)-1-méthyl-3-propyl-1H-pyrazolo-[4,3-d]pyrimidine, la (3-chloro-4-méthoxybenzyl)(1-méthyl-3-propyl-5-{[(pyridin-3-ylméthyl)amino]méthyl}-1H-pyrazolo[4,3-d]pyrimidin-7-yl)amine, le 5-{[7-(3-chloro-4-méthoxybenzylamino)-1-méthyl-3-propyl-1H-pyrazolo[4,3-d]pyrimidin-5-ylméthyl]amino}pentan-1-ol, le 3-{4-[7-(3-chloro-4-méthoxybenzylamino)-1-méthyl-3-propyl-1H-pyrazolo[4,3-d]pyrimidin-5-ylméthyl]pipérazin-1-yl]}propane-1,2-diol, la (3-chloro-4-méthoxybenzyl)[5-(4-diméthylaminopipéridin-1-yl-méthyl)-1-méthyl-3-propyl-1H-pyrazolo[4,3-d]pyrimidin-7-yl]amine, le 2-{[7-(3-chloro-4-méthoxybenzylamino)-1-méthyl-3-propyl-1H-pyrazolo[4,3-d]pyrimidin-5-ylméthyl]éthylamino}éthanol, la (3-chloro-4-méthoxybenzyl){1-méthyl-5-[(2-morpholin-4-yléthylamino)méthyl]-3-propyl-1H-pyrazolo[4,3-d]pyrimidin-7-yl}amine, le (2S,3S,4S,5R)-6-{[7-(3-chloro-4-méthoxybenzylamino)-1-méthyl-3-propyl-1H-pyrazolo[4,3-d]pyrimidin-5-ylméthyl]méthylamino}hexane-1,2,3,4,5-pentaol
ou l'un des sels physiologiquement acceptables de celui-ci.

10. Procédé de préparation d'un composé selon l'une des revendications 1 à 3, **caractérisé en ce qu'**une 5-halogénométhylpyrazolo[4,3-d]pyrimidine de formule générale (IIa) 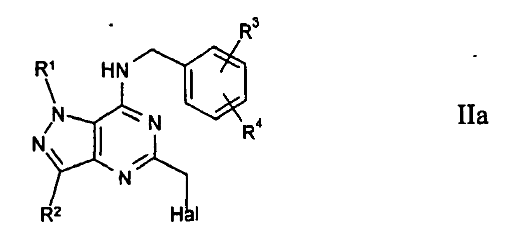 dans laquelle les substituants R¹ à R⁴ revêtent la signification indiquée selon la revendication 1, est réagi avec une alkylamine de formule générale (III)
HNR⁵R⁶ III
dans laquelle les substituants R⁵ et R⁶ revêtent la signification indiquée selon la revendication 1, et la substance obtenue est éventuellement purifiée.

11. Procédé de préparation d'un médicament, **caractérisé en ce qu'**un composé selon l'une des revendications 1 à 3 ou l'un des sels physiologiquement acceptables de celui-ci est mis sous une forme de dosage convenable, conjointement avec au moins un adjuvant ou excipient convenable.
